# FASCICULE DE BREVET EUROPEEN

(11) **EP 2 936 105 B1**
(45) Date de publication et mention de la délivrance du brevet: **16.10.2019**
(21) Numéro de dépôt: 13808023.9
(22) Date de dépôt: 18.12.2013
(51) Int. Cl.: G01M 3/20, G01D 3/08, G01N 33/00, G01N 27/407

(54) **DÉTECTEUR DE FUITE D'HYDROGÈNE**
WASSERSTOFFLECKDETEKTOR
HYDROGEN LEAKAGE DETECTOR

(30) Priorité: 19.12.2012 FR 1262345
(43) Date de publication de la demande: 28.10.2015
(73) Titulaire: Compagnie Générale des Etablissements Michelin, 63000 Clermont-Ferrand (FR)
(72) Inventeur: PAGANELLI, Gino, F-63040 Clermont-Ferrand Cedex 9 (FR); JEANRICHARD, Lionel, F-63040 Clermont-Ferrand Cedex 9 (FR)
(74) Mandataire: Roussy, Delphine
(86) Numéro de dépôt international: PCT/EP2013/077014
(87) Numéro de publication internationale: WO 2014/095950

(56) Documents cités:
- EP-A1- 1 505 385
- EP-A2- 2 522 974
- DE-A1-102011 103 248
- FR-A1- 2 412 055
- FR-A1- 2 966 241
- JP-A- S57 149 936
- JP-A- 2003 302 362
- JP-A- 2010 019 754
- US-A1- 2006 289 400
- US-A1- 2007 028 666
- US-A1- 2009 210 171
- US-A1- 2010 233 562
- US-A1- 2011 302 993

## Description

### DOMAINE DE L'INVENTION

La présente invention concerne les systèmes utilisant ou produisant de l'hydrogène. Plus particulièrement, elle vise les dispositifs permettant d'assurer la sécurité et la fiabilité de tels systèmes.

### ETAT DE LA TECHNIQUE

Depuis quelques années, l'utilisation de l'hydrogène comme source de combustible et d'énergie se développe dans de nombreux domaines. En effet, l'hydrogène présente de nombreux avantages, notamment celui d'être l'élément le plus abondant de la planète, et d'être un moyen de stockage d'énergie renouvelable.

Ce combustible universel peut être brûlé dans un moteur, ou utilisé dans une pile à combustible, pour alimenter des véhicules, des bâtiments, des centrales électriques, et plus généralement tout système nécessitant l'apport d'énergie. Outre les avantages précédemment mentionnés, l'hydrogène présente des taux d'efficacité bien supérieurs aux moteurs à essence, par exemple.

Un des sujets majeurs à traiter dans le cadre de développement de ces nouvelles solutions est celui de la sécurité. En effet, les gaz mis en oeuvre dans les différentes applications sont des gaz issus de l'atmosphère, mais également des gaz stockés sous pression. Or, on a constaté que des diffusions intempestives de ces gaz peuvent se produire, soit directement au niveau du moyen de stockage, soit lors d'un transfert de ce gaz du stockage vers le lieu d'utilisation. Certains de ces gaz étant inflammables, il est nécessaire de pouvoir détecter rapidement toute fuite, afin d'éviter des accidents dangereux pour les utilisateurs. En outre, afin de ne pas perturber inutilement le fonctionnement appareils, notamment les piles à combustible, il est utile de pouvoir éviter toute fausse détection de fuite qui conduirait, par exemple, à un arrêt de la pile non nécessaire.

Ainsi, on a vu se développer ces dernières années des capteurs de concentration de gaz, notamment d'hydrogène, dans des gaz de l'environnement des piles à combustible. De tels capteurs reposent sur une mesure la conductivité thermique à l'aide d'une unité sensible comportant une résistance chauffante dont l'échauffement ou le refroidissement dissipatif est fonction de la conductibilité thermique du gaz ambiant et donc en général de sa composition. Ces capteurs, relativement peu encombrants et simples à mettre en oeuvre, sont particulièrement bien adaptés aux mesures de la teneur d'un gaz en hydrogène en raison de la très forte conductibilité thermique de ce dernier gaz par rapport à celle de la plupart des gaz usuels auxquels il est susceptible d'être mélangé.

Par exemple, la demande publiée EP0291462 décrit ce principe de détection d'un gaz dans l'air sur la base de la variation de conductivité thermique. Plus précisément, ce document montre un micro-capteur de gaz mettant en oeuvre ce principe. L'élément sensible de ce micro-capteur est constitué d'une couche d'oxyde d'étain, obtenue par des techniques classiques d'attaque chimique et de dépôt par pulvérisation à partir d'un substrat de silicium.

On a envisagé d'utiliser un tel capteur de concentration d'hydrogène pour détecter les fuites d'hydrogène dans l'environnement de systèmes utilisant l'hydrogène comme combustible. En effet, il est possible d'utiliser un microcontrôleur permettant de générer un signal analogique représentatif de la concentration en hydrogène mesurée, et ainsi d'alerter en cas d'augmentation forte et imprévue de cette concentration. Toutefois, on a constaté une faille de sécurité dans un tel dispositif, puisque le signal correspondant à une mesure de concentration nulle consécutive à une absence effective d'hydrogène dans l'air est similaire au signal correspondant à une mesure de concentration nulle consécutive à une défaillance du capteur et/ou d'un autre élément du dispositif.

Par conséquent, les dispositifs existants n'offrent pas une fiabilité suffisante puisqu'une fuite d'hydrogène peut se produire sans être détectée. La présente invention vise à proposer un dispositif permettant de répondre à cette exigence de fiabilité.

On connait par ailleurs le document US 2207/0028666 qui divulgue une unité de détection de concentration d'hydrogène dans l'air à deux capteurs et des moyens pour afficher une information analogique de concentration issue d'un des capteurs lorsqu'il qui fonctionne normalement ou un message d'erreur lorsque les capteurs sont défaillants. Un tel dispositif s'avère complexe et onéreux.

### EXPOSE DE L'INVENTION

Ainsi, la présente invention concerne un détecteur de fuite d'hydrogène muni de moyens permettant de vérifier, en temps réel, le bon fonctionnement du dispositif de détection. De manière plus précise, la présente invention concerne un détecteur de fuite d'hydrogène selon la revendication 1.

Par ailleurs, dans une réalisation avantageuse, le microcontrôleur comprend des moyens de vérification de l'intégrité d'un ou plusieurs composants du capteur. Ainsi, la présence de ce signal analogique de bon fonctionnement de l'organe de suivi de condition permet d'alerter un utilisateur d'une défaillance d'un élément du dispositif de détection.

En outre, dans une autre réalisation de l'invention, le microcontrôleur comprend des moyens de vérification de cohérence de la mesure de concentration avec l'information analogique de concentration générée.

Dans une autre réalisation de l'invention, le signal analogique de bon fonctionnement est un signal normalement haut. Ainsi, lorsque le dispositif fonctionne correctement, un signal haut est émis, et lorsqu'une défaillance du dispositif est détectée, un signal bas, ou nul, est émis. Ceci permet, en outre, qu'une panne d'alimentation du dispositif soit également interprétée comme une défaillance, puisque dans ce cas le signal analogique ne serait pas émis, ce qui correspond à un signal nul.

Selon l'invention, le détecteur, ou l'organe de suivi de condition, comprend une unique sortie analogique. Dans ce cas, le signal représentatif de la concentration en gaz mesurée, et le signal de bon fonctionnement doivent être émis sur une unique sortie. Par conséquent, il est utile, dans cette configuration, que le signal analogique de bon fonctionnement soit un signal normalement haut émis à intervalles réguliers. Ceci permet d'émettre deux informations distinctes sur une même sortie, sans que ces deux informations ne soient confondues.

### BREVE DESCRIPTION DES FIGURES

D'autres caractéristiques et avantages de l'invention ressortent de la description faite ci-après en référence aux dessins annexés qui montrent, à titre d'exemples non limitatifs, des formes de réalisation de l'objet de l'invention.

La figure 1 est un schéma très simplifié de la réalisation d'un détecteur de concentration d'hydrogène avec son électronique de traitement dans un exemple d'application, en l'occurrence avec une pile à combustible.

La figure 2 montre l'évolution d'un signal analogique délivré par un capteur selon l'invention.

### DESCRIPTIOND'UN OU PLUSIEURS EXEMPLES DE REALISATION

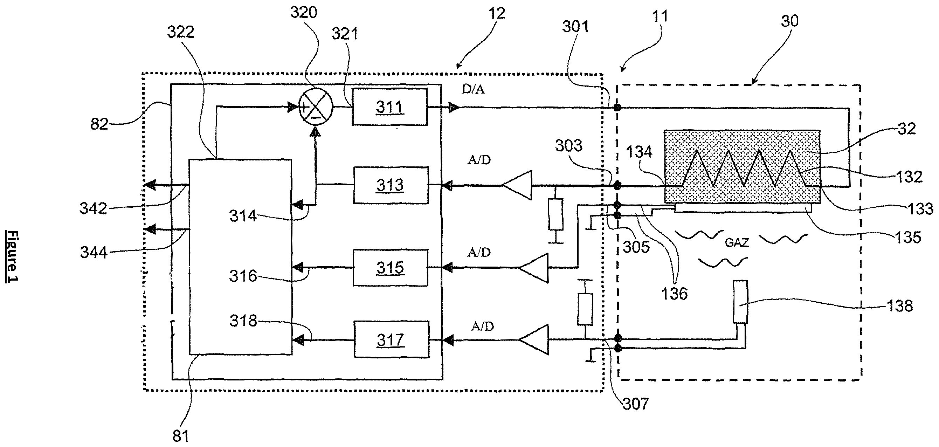

La figure 1 montre un schéma d'un capteur d'hydrogène 11 qui comprend une plaquette 32 de circuits sensibles composée d'un substrat en forme de plaque ou de film, par exemple en silicium. Le substrat est revêtu d'une résistance électrique de chauffage 132, intégrée ou déposée, alimentée à partir de bornes d'alimentation 133 et 134 par une source de tension contrôlée par l'unité de traitement 12. Une des faces de la plaquette 32 comporte un capteur de température 135, par exemple formé par une couche thermoresistive dite PT100, relié par un jeu de conducteurs 136 à l'unité de traitement 12 pour fournir les signaux correspondant à la température instantanée de la plaquette d'unité sensible 32, sous la double action du chauffage et de la dissipation calorifique dans le gaz ambiant. En outre, le capteur de concentration d'hydrogène 11 intègre une sonde de température 138, par exemple une sonde PT 100, pour connaître la température du gaz ambiant. La partie physique de tels capteurs est par exemple décrite dans les documents de brevet EP 0291 462 B1 du 11 mai 1988 et EP 0 501 089 A1 du 25 février 1991.

L'unité de traitement 12 du capteur de concentration d'hydrogène 11 comprend un module numérique 82 relié par quatre lignes de signaux à l'unité sensible 32. Le module numérique 82, schématisé sur la figure 1, est une représentation synoptique des fonctions d'un algorithme implanté dans un microcontrôleur et qui comprend un module de calcul 81 des grandeurs de sortie du capteur de concentration d'hydrogène 11.

La première ligne 301 à la sortie de l'unité de traitement 12 contrôle, via un convertisseur numérique/analogique, la tension appliquée sur la résistance de chauffage 132 du capteur de la plaquette 32. La valeur de la tension peut être commandée à partir d'un étage de régulation de la puissance de chauffage 311 dans l'unité de commande 80. Dans la suite on considère que la résistance de chauffage est alimentée en mode régulation de puissance, cependant d'autres modes d'alimentation de la résistance de chauffage 132 sont possibles, par exemple régulation de tension ou régulation de courant.

La deuxième ligne 303 reçoit le signal de tension image du courant traversant la résistance de chauffage 132. Cette information est convertie par un convertisseur numérique/analogique A/D à l'entrée de l'unité de traitement 12 pour être exploitée par un circuit de contrôle 313 de la puissance de chauffe réellement dissipée dans la résistance 132 du capteur de concentration d'hydrogène 11. On voit sur le schéma que cette information, envoyée à un comparateur 320 dans le module numérique 82 est comparée à une valeur de consigne produite en sortie 322 du module de calcul 81. Dans le module numérique 82, le produit de la comparaison de ces deux grandeurs contrôle l'entrée 321 du régulateur de puissance de chauffe 311. Le chauffage de la plaquette 32 de l'unité sensible 30 est donc généré par une boucle de contre réaction numérique à partir de la consigne de puissance de chauffe affichée à chaque instant à la sortie 322 du module numérique 82. Dans le cas d'une alimentation à tension constante, le régulateur de puissance est remplacé par un régulateur de tension. Même dans le cas d'une régulation de tension, il est important que la puissance réellement dissipée dans la résistance de chauffage soit mesurée par le module numérique 82 pour garantir une bonne précision du calcul de la concentration d'hydrogène.

La troisième ligne 305 permet d'envoyer à une entrée de l'unité de traitement 12 le signal analogique de sortie du capteur de température 135 de la plaquette 32. Après conversion analogique numérique ce signal est exploité par un calculateur de température 315 dans le module numérique 82 qui alimente à son tour le module de calcul 81 avec cette information.

Enfin la quatrième ligne 307 correspond à une entrée de l'unité de traitement 12 qui reçoit la tension de sortie de la sonde de température 138 (température ambiante) et, après conversion analogique numérique, transmet l'information à un calculateur de température 317 dans le module numérique pour l'afficher à l'entrée du module de calcul 81.

Le module de calcul 81 qui reçoit donc, sur ses entrées 314, 316, et 318, les informations de puissance effective de chauffage du capteur, de température de la plaquette 32 et de température ambiante de la sonde de température 138 permet de déterminer : la concentration en hydrogène et la température. L'humidité, quant à elle, peut être déduite d'autres mesures ou être mesurée à partir d'un capteur d'humidité spécifique non représenté.

Le capteur comprend une sortie analogique 342 sur laquelle on retrouve la concentration en hydrogène représentée par un signal entre 0 et 5 volts, et une sortie CAN 344 sur laquelle on retrouve la concentration en hydrogène, avec en plus l'humidité et la température.

Par ailleurs, un capteur selon l'invention comprend, dans le module 82, des moyens pour émettre un signal d'autotest permettant de vérifier, en temps réel, le bon fonctionnement du capteur. Ainsi, ces moyens d'autotest comprennent des moyens permettant de lister toutes les défaillances possibles du capteur. Par ailleurs, le dispositif comprend également une instrumentation spécifique de l'ensemble des éléments de détection, permettant de tester en temps réel les fonctionnalités.

Lorsque les moyens d'autotest déterminent que l'ensemble des fonctionnalités du capteur est active, un signal de bon état de fonctionnement est émis sur la sortie analogique 342, c'est-à-dire sur la même sortie que le signal représentant la concentration en hydrogène. La défaillance d'une seule fonctionnalité entraîne l'émission d'un signal « not ok ». De manière préférentielle, le signal de bon fonctionnement est un signal haut. De manière préférentielle encore, le signal de bon fonctionnement correspond à des artefacts émis régulièrement sur la sortie 342, et qui permettent donc d'indiquer le bon fonctionnement du capteur sans toutefois gêner la transmission de l'information importante, à savoir la concentration en hydrogène.

Le choix d'un signal normalement haut résulte de la volonté de pouvoir également détecter une panne d'énergie dans le détecteur. En effet, une panne d'énergie conduirait à une absence de signal, qui serait ainsi interprétée comme un dysfonctionnement.

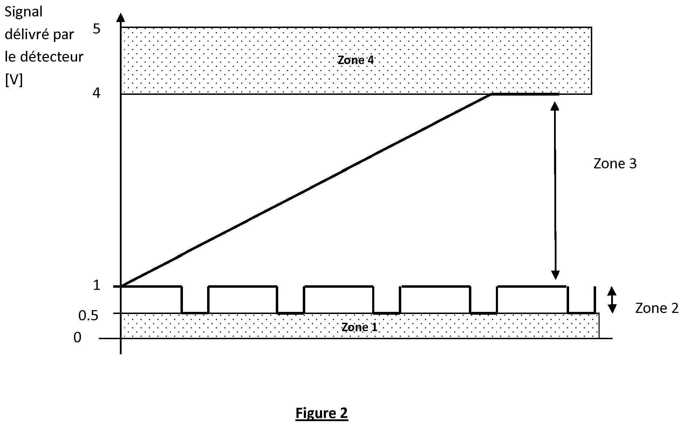

La figure 2 montre ainsi un exemple de signal analogique émis sur la sortie 342 du détecteur. On constate que ce signal est délivré sur une plage de 0 à 5 volts.

Le signal d'autotest est un signal périodique en créneaux, avec une valeur basse égale, par exemple, à 0.5 volts, et une valeur haute égale à 1 volt.

Le signal de détection d'hydrogène est un signal proportionnel. Ainsi, une fuite d'hydrogène est représentée, sur la sortie analogique, par un signal proportionnel, entre 1 et 4 volts. La valeur 1 volt correspond à une concentration de 0% d'hydrogène dans l'air, et la valeur 4 volts correspond à une concentration de 4%.

Ainsi, lorsque le signal délivré par le détecteur est situé entre 0 et 0.5 volts, c'est le signe d'un comportement anormal du détecteur (zone 1 sur la figure 2).

Lorsque le signal délivré est situé entre 0.5 volts et 1 volts, (zone 2 sur la figure 2) cela signifie que le détecteur fonctionne normalement, et qu'aucune fuite d'hydrogène n'est détectée.

Lorsque le signal délivré est situé entre 1 et 4 volts (zone 3 sur la figure 2), cela signifie qu'une fuite d'hydrogène est détectée.

Lorsque le signal délivré est situé entre 4 et 5 volts, c'est le signe d'un comportement anormal du détecteur (zone 4 sur la figure 2).

Ainsi, un arrangement selon la présente invention permet de garantir une détection efficace d'une fuite d'hydrogène et d'un dysfonctionnement du détecteur. En effet, le choix d'un signal autotest périodique permet de prévenir le cas d'un signal qui resterait figé, par exemple à une valeur non nulle. En effet, un tel signal figé pourrait donner l'impression d'un fonctionnement normal du détecteur alors même qu'un dysfonctionnement pourrait avoir survenu.

Par ailleurs, le choix d'une valeur basse du signal périodique non nulle permet de garantir une détection immédiate d'un dysfonctionnement du détecteur. En effet, si le signal d'autotest avait une valeur basse nulle, cela signifierait qu'un signal nul délivré en sortie du capteur ne serait pas nécessairement représentatif d'un dysfonctionnement du capteur. Il faudrait donc attendre le passage à la valeur haute du signal pour avoir la confirmation de l'état de fonctionnement du détecteur. Or, un tel délai d'attente peut s'avérer dangereux dans le cas d'une fuite d'hydrogène massive.

On a donc décrit ici un dispositif de détection de fuite d'hydrogène permettant d'effectuer une détection fiable. En effet, ce dispositif de détection permet de distinguer le cas d'une mesure d'hydrogène nulle parce qu'il n'y a effectivement pas d'hydrogène dans l'air du cas d'une mesure de concentration nulle parce que le détecteur est défaillant. Ceci permet d'assurer une sûreté de fonctionnement nécessaire pour l'utilisation d'un réacteur électrochimique, par exemple sur un véhicule motorisé.

Par ailleurs ce dispositif de détection est relativement facile à installer puisque l'utilisation d'une unique sortie analogique pour émettre le signal de concentration en hydrogène et le signal de bon fonctionnement permet de limiter le nombre de sortie et donc le nombre de câblages à effectuer. Ceci permet également de limiter les coûts supplémentaires d'un tel dispositif.

L'invention n'est bien entendu pas limitée aux exemples décrits et représentés et diverses modifications peuvent y être apportées sans sortir de son cadre défini par les revendications annexées.

## Revendications

1. Détecteur de fuite d'hydrogène, comportant:
un capteur (11) sensible à la concentration d'hydrogène dans l'air, le dit capteur comprenant une unité sensible (30) exposée directement à la concentration in situ d'hydrogène, et
un microcontrôleur (82) comprenant:
- des moyens pour générer et transmettre, à partir de la mesure de concentration, une information analogique de concentration, et
- des moyens pour générer et transmettre un signal analogique de bon fonctionnement du détecteur, et
une unique sortie analogique,
**caractérisé en ce que** le microcontrôleur comprend des moyens de vérification de l'intégrité d'un ou plusieurs composants du microcontrôleur et que l'unique sortie analogique est apte à représenter deux informations distinctes : le signal analogique de bon fonctionnement du capteur (11) et l'information analogique de concentration mesurée par le capteur (11).

2. Détecteur selon la revendication 1, **caractérisé en ce que** ledit microcontrôleur comprend des moyens de vérification de l'intégrité d'un ou plusieurs composants du capteur.

3. Détecteur selon l'une des revendications 1 ou 2, **caractérisé en ce que** ledit microcontrôleur comprend des moyens de vérification de cohérence de la mesure de concentration avec l'information analogique de concentration générée.

4. Détecteur selon l'une des revendications 1 à 3, **caractérisé en ce que** le signal analogique de bon fonctionnement est un signal normalement haut.

5. Détecteur selon la revendication 4, **caractérisé en ce que** le signal analogique de bon fonctionnement est un signal normalement haut émis à intervalles réguliers sur la sortie analogique du microcontrôleur.

6. Détecteur selon l'une des revendications précédentes, **caractérisé en ce que** le signal analogique de bon fonctionnement est un signal périodique de type créneau.

7. Détecteur selon la revendication précédente, **caractérisé en ce que** le signal analogique périodique à une valeur inférieure non nulle.

## Patentansprüche

1. Wasserstoffleckdetektor, der aufweist:
einen Sensor (11), der auf die Wasserstoffkonzentration in der Luft anspricht, wobei der Sensor eine empfindliche Einheit (30) aufweist, die direkt der Wasserstoffkonzentration vor Ort ausgesetzt ist, und
einen Mikrocontroller (82), der enthält:
- Einrichtungen, um ausgehend von der Konzentrationsmessung eine analoge Konzentrationsinformation zu erzeugen und zu übertragen, und
- Einrichtungen, um ein analoges Signal einwandfreien Betriebs des Detektors zu erzeugen und zu übertragen, und
einen einzigen analogen Ausgang,
**dadurch gekennzeichnet, dass** der Mikrocontroller Einrichtungen zur Überprüfung der Integrität eines oder mehrerer Bestandteile des Mikrocontrollers enthält, und dass der einzige analoge Ausgang zwei unterschiedliche Informationen darstellen kann: das analoge Signal einwandfreien Betriebs des Sensors (11) und die vom Sensor (11) gemessene analoge Konzentrationsinformation.

2. Detektor nach Anspruch 1, **dadurch gekennzeichnet, dass** der Mikrocontroller Einrichtungen zur Überprüfung der Integrität eines oder mehrerer Bestandteile des Sensors enthält.

3. Detektor nach einem der Ansprüche 1 oder 2, **dadurch gekennzeichnet, dass** der Mikrocontroller Einrichtungen zur Überprüfung der Kohärenz der Konzentrationsmessung mit der erzeugten analogen Konzentrationsinformation enthält.

4. Detektor nach einem der Ansprüche 1 bis 3, **dadurch gekennzeichnet, dass** das analoge Signal einwandfreien Betriebs ein normalerweise hohes Signal ist.

5. Detektor nach Anspruch 4, **dadurch gekennzeichnet, dass** das analoge Signal einwandfreien Betriebs ein normalerweise hohes Signal ist, das in gleichmäßigen Abständen am analogen Ausgang des Mikrocontrollers emittiert wird.

6. Detektor nach einem der vorhergehenden Ansprüche, **dadurch gekennzeichnet, dass** das analoge Signal einwandfreien Betriebs ein periodisches Signal der Art Rechtecksignal ist.

7. Detektor nach dem vorhergehenden Anspruch, **dadurch gekennzeichnet, dass** das periodische analoge Signal einen unteren Wert ungleich Null hat.

## Claims

1. Hydrogen leak detector, having:
a sensor (11) sensitive to the concentration of hydrogen in the air, the said sensor comprising a sensitive unit (30) exposed directly to the in situ concentration of hydrogen, and
a microcontroller (82) comprising:
- means for generating and transmitting analogue concentration information on the basis of the concentration measurement, and
- means for generating and transmitting an analogue signal of correct operation of the detector, and
- a single analogue output,
**characterised in that** the microcontroller comprises means for verifying the integrity of one or more components of the microcontroller and **in that** the single analog output is able to represent two separate items of information: the analog signal of correct operation of sensor (11) and the analog information of the gas concentration measured by sensor (11).

2. System according to Claim 1, **characterized in that** the said microcontroller comprises means for verifying the integrity of one or more components of the sensor.

3. System according to either of Claims 1 and 2, **characterized in that** the said microcontroller comprises means for verifying the consistency of the concentration measurement with the analogue concentration information generated.

4. System according to one of Claims 1 to 3, **characterized in that** the analogue signal of correct operation is a normally high signal.

5. System according to Claim 4, **characterized in that** the analogue signal of correct operation is a normally high signal emitted at regular intervals on the analogue output of the microcontroller.

6. System according to one of the preceding claims, **characterized in that** the analogue signal of correct operation is a periodic signal of the squarewave type.

7. System according to the preceding claim, **characterized in that** the periodic analogue signal has a nonzero lower value.
